# EUROPÄISCHE PATENTSCHRIFT

(11) **EP 0 682 945 B1**
(45) Veröffentlichungstag und Bekanntmachung des Hinweises auf die Patenterteilung: **09.06.1999**
(21) Anmeldenummer: 95890095.3
(22) Anmeldetag: 18.05.1995
(51) Int. Cl.: A61K 9/22

(54) **Orale Retard-Präparation**
Oral sustained release preparation
Préparation orale à effet retard

(30) Priorität: 18.05.1994 AT 102794
(43) Veröffentlichungstag der Anmeldung: 22.11.1995
(73) Patentinhaber: Lannacher Heilmittel Ges.m.b.H., A-8502 Lannach (AT)
(72) Erfinder: Posch, Werner, Dr., A-8071 Vasoldsberg (AT); Reiter, Franz Josef, Mag. pharm. Dr., A-8044 Graz (AT)
(74) Vertreter: Haffner, Thomas M., Dr.

(56) Entgegenhaltungen:
- EP-A- 0 338 383
- WO-A-91/19483
- WO-A-91/19486
- US-A- 4 708 874
- US-A- 4 792 452
- US-A- 5 229 135

## Beschreibung

Die Erfindung bezieht sich auf eine orale Retard-Präparation für die verzögerte Abgabe eines Wirkstoffes in Form von Tabletten unter Verwendung von magensaftresistenten Polyacrylaten.

Orale Retard-Präparationen von Arzneimittel bewirken, daß der Wirkstoff aus dem Arzneimittel im Gastrointestinaltrakt über einen längeren Zeitraum kontrolliert freigesetzt wird. Auf diese Art und Weise sollen die Häufigkeit der Einnahmen reduziert und gleichmäßige Plasmaspiegel aufrechterhalten werden, um einen gleichmäßigen pharmakologischen Effekt zu erzielen. Die therapeutisch wirksame Konzentration eines Medikamentes soll bis zu 12 Stunden erhalten bleiben.

Um über einen längeren Zeitraum eine therapeutisch wirksame Konzentration eines oral verabreichten Arzneimittels zu erreichen, sind folgende Faktoren ausschlaggebend:
- die Wirkstofffreisetzung aus der Arzneiform
- die Resorption des Wirkstoffes an der Schleimhaut des Magen-Darm-Kanals
- der First-pass-Effekt in der Leber.

Diese Faktoren sind durch eine entsprechende Arzneimittelgalenik beeinflußbar.

Aus der WO-A1-91/16402 sind pH-abhängige Polymere und unter anderem pH-abhängige Polyacrylate als Material einer Beschichtung bzw. im Magensaft unlösbaren Membran bekannt geworden. Derartige Beschichtungen dienen hierbei dazu, eine Freisetzung von Wirkstoffen im Magen zu verhindern und erst im Darmtrakt zuzulassen.

Die Erfindung zielt nun darauf ab, feste pharmazeutische Zubereitungen zu schaffen, bei welchen der Wirkstoff über einen langen Zeitraum im Bereich des Verdauungstraktes gleichmäßig und vollständig abgegeben wird, wobei die Wirkstoffreisetzung möglichst bald nach der Einnahme des Präparates ermöglicht werden soll. Zur Lösung dieser Aufgabe besteht die erfindungsgemäße feste orale Präparation im wesentlichen darin, daß als Wirkstoff Morphin, Diltiazem, Codein, Dihydrocodein, Dramatol oder Propoxyphen vorliegt und daß eine Mischung aus pH-abhängig und pH-unabhängig retardierenden Polyacrylaten in der Matrix enthalten ist, wobei 10 bis 60 Gew.% pH-abhängig und 3 bis 30 Gew.% pH-unabhängig retardierendes Polyacrylat im Präparat vorliegt. Dadurch, daß pH-unabhängig und ph-abhängig retardierende Polyacrylate gemeinsam in der Matrix verwendet werden, wird die Möglichkeit geschaffen, die Abgabe des Wirkstoffes im Magen ebenso wie im Darmtrakt zu verzögern und über den Zeitraum zu vergleichmäßigen. Es kann sichergestellt werden, daß die Abgabe des Wirkstoffes innerhalb von zumindest 8 Stunden gleichmäßig und vollständig erfolgt, wofür erfindungsgemäß die Ausbildung so getroffen ist, daß 10 bis 60 Gew.% pH-abhängig und 3 bis 30 Gew.% pH-unabhängig retardierendes Polyacrylat vorliegt.

Die Verwendung derartiger Polyacrylate stellt hierbei sicher, daß der Wirkstoff zur Gänze im Körper resorbiert werden kann und nicht, wie bei anderen unlöslichen Trägern oder Matrixbestandteilen an ungelösten Teilen adsorbiert oder in solchen okkludiert wieder ausgeschieden werden kann. Die Dosierung des Wirkstoffes wird dadurch vereinfacht, und die Wahl pH-abhängig und pH-unabhängig retardierender Polymere der gleichen Art, nämlich Polyacrylate in der Matrix, ermöglicht eine einfache und weitgehend lineare Einstellung des Lösungs- und damit Freisetzungsverhaltens über die gesamte Verweilzeit im Gastro-Intestinaltrakt.

Eine besonders gleichmäßige und über den geforderten Zeitraum auch vollständige Abgabe des Wirkstoffes kann dadurch sichergestellt werden, daß 2 bis 20 Gew.% und vorzugsweise 3 bis 6 Gew.% Hydroxypropylmethylcellulose im Präparat vorliegt.

Bei der Wahl des Wirkstoffes ist prinzipiell darauf zu achten, daß eine Fällung aufgrund des jeweils vorherrschenden pH-Wertes vermieden wird und eine entsprechend wasserlösliche Form des Wirkstoffes vorliegt. Die erfindungsgemäße Retardpräparation hat sich daher für Präparate bewährt, bei welchen als Wirkstoff Morphin oder Diltiazem, Codein, Dihydrocodein, Dramatol oder Propoxphen in jeweils gut wasserlöslicher Form, beispielsweise in Form von Salzen vorliegt.

Das erfindungsgemäße Verfahren zur Herstellung eines festen oralen Retardpräparates ist im wesentlichen dadurch gekennzeichnet, daß zu dem aus Wirkstoff, ggf. Füllstoff und Granulierflüssigkeit erhaltenen Granulat 10 % bis 60 % ph-abhängig retardierendes Polyacrylat, 3 % bis 30 % ph-unabhängig retardierendes Polyacrylat, und 2 % bis 20 % Hydroxypropylmehtylcellulose zugemischt werden, worauf unter Verwendung üblicher Tablettierhilfsstoffe zu Tabletten verpreßt wird. Tabletten können hiebei ggf. Füllstoffe, Bindemittel, Gleitmittel, Granuliermittel und andere übliche Zusätze für pharmazeutische Zubereitungen enthalten, wobei die für die vorliegende Erfindung wesentlichen Anteile die jeweils zugemischten Polyacrylate sowie die Anteile an Hydroxypropylmehtylcellulose sind.

Zur Geschmacksabdeckung kann mit Vorteil so vorgegangen werden, daß die verpreßten Tabletten mit einem rasch zerfallenden Film überzogen werden.

Eine über die Zeit vollständig gleichmäßige und vollständige Wirkstoffabgabe kann so erzielt werden, daß das Verhältnis pH-unbhängig retardierendes zu pH-abhängig retardierendem Polyacrylat so eingestellt wird, daß in der Zeiteinheit im Magen- und und im Darmtrakt die gleiche Wirkstoffmenge resorbiert wird.

Die Wirkungsweise der Polyacrylate in der Matrix ist in bezug auf Einstellbarkeit und Vollständigkeit der Wirkstoffabgabe der bekannten Verwendung von Coatings deutlich überlegen. Schutzüberzüge aus magensaftresistenten Polyacrylaten erlauben eine Verzögerung der Wirkstoffreisetzung dadurch, daß diese im Magen verhindert wird, wodurch aber eine konstante Wirkstoffreisetzung in der Zeit im Magen und im Darm nicht erzielt werden kann.

Die Erfindung wird nachfolgend anhand von Ausführungsbeispielen näher erläutert. Weitere Anwendungsmöglichkeiten kann der Fachmann ohne weiteres ableiten.

### AUSFÜHRUNGSBEISPIELE:

### Beispiel 1:

### Herstellung des Granulates:

In einen geeigneten Mixer werden eingewogen

| | |
|---|---|
| Morphin, HCl x 3H20 | 15,00 kg |
| Lactose-monohydrat | 12,00 kg, |

durchmischt und mit einer Mischung aus

| | |
|---|---|
| Wasser, gereinigt | 5,00 kg |
| Poly(ethylacrylat, methylmethacrylat) 2:1 (Eudragit NE 30 D) | 10,00 kg |

granuliert und anschließend bei 40-45° C getrocknet.

### Herstellung der Tablettiermischung:

In einem geeigneten Mischer werden eingewogen:

| | |
|---|---|
| Wirkstoff-Granulat | 30,00 kg |
| Poly(ethylacrylat, methacrylsäure) 1:1 (Eudragit L 100-55) | 34,00 kg |
| Poly (ethylacrylat, methylmethacrylat, trimethylammonium ethylmethacrylatchlorid) 1:2:0,1 Eudragit RSPM) | 7,50 kg |
| Hydroxypropylmethylcellulose 4000 | 3,00 kg |
| Magnesimstearat | 0,50 kg |

und homogen gemischt.

### Tablettierung:

Die fertige Tablettiermischung wird auf einer geeigneten Rundlauftablettierpresse im spezifizierten Format verpreßt (runde, bikonvex geformte Tabletten mit einem Durchmesser von 7 mm, einer Höhe von ca 4 mm und einem Gewicht von 150 mg).

### Film coating :

Die entstaubten Tablettenkerne werden in einem konventionellen Dragierkessel mit einer schmelzverfallenden Filmhülle überzogen.

### Die Filmhüllenmasse besteht aus:

| | |
|---|---|
| Titan (IV) oxid | 785,0 g |
| Polyethylenglykol 6000 | 715,0 g |
| Talkum | 1.952,0 g |
| Hydroxypropylmethylcellulose (Methocel E5) | 570,0 g |
| Antischaumemulsion | 3,0 g |
| Poly(ethylacrylat, methylmethacrylat) 2:1 (Eudragit NE30D, 30%ige Suspension) | 2.150,0 g |
| LMF Lackgelb E104/110 | 255,0 g |
| LMF Gelborange E110 | 75,0 g |
| Wasser gereinigt (wird aus dem Prozeß durch Trocknung wieder entfernt | 10.285,0 g |

Die in vitro Wirkstofffreisetzungsprüfung der in der beschriebenen Weise hergestellten Filmtabletten wurde nach den Vorgaben der USP XXII untersucht. Nach zwei Stunden im künstlichen Magensaft (0,1 N Salzsäure pH 1,2) wird das Medium auf künstlichen Darmsaft gewechselt (pH Einstellung auf pH 6,8). Bei den durchgeführten Untersuchungen gaben die Filmtabletten den Wirkstoff (Morphin.HCl) innerhalb der in folgenden angegebenen Zeiten folgende Mengen ab:

| Nach | Abgegebene Menge an Morphin HCl in % der Gesamtdosierung |
|---|---|
| 1 Stunde | 22 % |
| 2 Stunden | 17 % |
| 4 Stunden | 31 % |
| 6 Stunden | 20 % |
| 8 Stunden | 10 % |
| | Insgesamt 100 % |

### Klinische Daten zu Morphin-Filmtabletten gemäß Beispiel 1:

Die perorale Gabe von Morphin ist heute die Standardtherapie zur Behandlung starker Schmerzen. Eine intravenöse Verabreichung wird nur in jenen Fällen durchgeführt, in denen der Patient oral nichts aufnehmen kann, darf oder will. Oral muß Morphin in Form einer wäßrigen Lösung etwa alle 4 Stunden verabreicht werden, um permanente Schmerzfreiheit zu erreichen.

Auf mehr als acht Stunden ausgelegte Retardformen sind für den Patienten von großem Vorteil und sollen ihn über diese Zeit tatsächlich schmerzfrei halten.

In einer Untersuchung zu Bioverfügbarkeit der oben dargestellten Arzneimittelformulierung von Morphinhydrochlorid wurden diese mit einer wäßrigen Lösung von Morphinhydrochlorid in gleicher Dosierung (30 mg) an Probanden untersucht. Dabei erhielt man folgende Ergebnisse im Mittel:

| Retardtablette | wäßrige Lösung Vendal retard | |
|---|---|---|
| Cₘₐₓ [ng/ml] | 79,15 | 331,00 |
| Tₘₐₓ [h] | 3,00 | 1,00 |
| AUC [ngxh/ml] | 852,56 | 1485,08 |
| HVD [h] | 8,30 | 2,84 |
| rel BA [%] | 57,40 | 100,0 |
| MRT [h] | 9,00 | 4,64 |

### Legende:

- Cₘₐₓ [ng/ml]: Maximale Plasmakonzentration
- Tₘₐₓ [h]: Zeit bis zum Erreichen von Cₘₐₓ
- AUC [ngxh/ml]: Fläche unter der Konzentration-Zeit-Kurve
- HVD [h]: Zeit, in der Cₘₐₓ/2 gehalten wird.
- rel BA [%]: relative Bioverfügbarkeit
- MRT [h]: mittlere Verweilzeit

Morphin muß bei oraler Anwendung etwa 3 mal so hoch dosiert werden wie bei parenteraler, also in einer Dosis von 30 mg statt 10 mg gegeben werden, um eine gleiche analgetische Wirkung zu erzielen; die Bioverfügbarkeit beträgt im Mittel 38 %. Die (im Vergleich zur wäßrigen Lösung) mit der Retardformulierung erreichten, wesentlich länger andauernden therapeutischen Morphinplasmaspiegel gewährleisten eine lange (bis zu 12 Stunden) Schmerzfreiheit der Patienten.

### Beispiel 2:

### Herstellung des Granulates:

In einem geeigneten Mixer werden eingewogen:

| | |
|---|---|
| Diltiazem.HCl | 18,00 kg |
| Lactose-Monohydrat | 12,00 kg |

durgemischt und mit einer Mischung aus

| | |
|---|---|
| Wasser, gereinigt | 1,90 kg |
| Poly(ethylacrylat, methylmethacrylat) 2:1 (Eudragit NE 30 D) | 3,00 kg |

granuliert und anschließend bei 40 - 45 °C getrocknet.

### Herstellung der Tablettiermischung:

In einem geeigneten Mischer werden eingewogen:

| | |
|---|---|
| Wirkstoff-Granulat | 30,90 kg |
| Poly(ethylacrylat, methacrylsäure) 1:1 (Eudragit L 100-55) | 7,95 kg |
| Poly(ethylacrylat, methylmethacrylat, trimethylammonium-methylmethacrylatchlorid (Eudragit RSPM) | 1,50 kg |
| Hydroxypropylmethylcellulose 4000 | 1,50 kg |
| Magnesiumstearat | 0,15 kg |

und homogen gemischt.

### Tablettierung:

Die fertige Tablettiermischung wird auf einer geeigneten Rundlauftablettierpresse im spezifizierten Format verpreßt (runde, bikonvex geformte Tabletten mit einem Durchmesser von 11 mm, einer Höhe von ca. 4 mm und einem Gewicht von 420 mg.

### Filmcoating:

Die entstaubten Tablettenkerne werden in einem konventionellen Dragierkessel mit einer schmelzverfallenden Filmhülle überzogen.

Die Filmhülle besteht aus:

| | |
|---|---|
| Titan(IV)dioxid | 123,0 g |
| Polyethylenglycol 6000 | 276,0 g |
| Talkum | 1142,0 g |
| Hydroxypropylmethylcellulose (Methocel E5) | 230,0 g |
| Antischaumemulsion | 1,8 g |
| Poly(ethylacrylat, methylmethacrylat) 2:1 (Eudragit NE 30 D, 30 % Suspension) | 2270,0 g |
| Wasser gereinigt (wird aus dem Prozeß durch Trocknung wieder entfernt | 5034,0 g |

Die in vitro Wirkstofffreisetzung der in der beschriebenen Weise hergestellten Filmtabletten wurde nach den Vorgaben der USP XXII untersucht.

Nach zwei Stunden im künstlichen Magensaft (0,1 N Salzsäure pH 1,2) wird das Medium auf künstlichen Darmsaft gewechselt (Einstellung des pH-Wertes auf 6,8).

Bei den durchgeführten Untersuchungen gaben die Filmtabletten innerhalb der angegebenen Zeiten folgende Mengen des Wirkstoffes (Diltiazem.HCl) ab:

| Nach | Abgegebene Menge an Diltiazem.HCl in % der Gesamtdosierung |
|---|---|
| 1. Stunde | 32 % |
| 2. Stunde | 16 % |
| 4. Stunde | 16 % |
| 6. Stunde | 18 % |
| 8. Stunde | 18 % |
| | Gesamt: 100 % |

### Klinische Daten zu Diltiazem 180 mg-Filmtabletten gemäß Beispiel 2:

Nach peroraler Gabe wird Diltiazem praktisch vollständig resorbiert.

Die Bioverfügbarkeit liegt wegen eines deutlichen First-Pass-Effektes dagegen nur bei etwa 50 %. Die Substanz mit einer Plasmahalbwertszeit von 4 bis 5 Stnden wird im Organismus desacetyliert und zudem oxidativ O- und N-demethyliert. Auch werden Phase II-Reaktionsprodukte (Konjugate der phenolischen Metabolite) beschrieben.

Die Elimination erfolgt fast ausschließlich in Fom von Metaboliten renal und daneben auch biliär.

In einer Untersuchung zur Bioverfügbarkeit von Diltiazem Hydrochlorid als Retardformulierung wurde diese in einer Dosierung von 180 mg an 16 Probanden untersucht.

Dabei erhielt man folgende Ergebnisse im Mittel:

| | Retardtablette Diltiazem retard |
|---|---|
| Cₘₐₓ [ng/mg] | 119,02 |
| Cₛₛ min [ng/ml] | 15,03 |
| AUCₛₛ, 0-24 [ngxh/ml] | 1379,43 |
| HVD [h] | 9,66 |
| PTF [%] | 176,96 |

### Zeichenerklärung:

- Cₘₐₓ: Maximale Plasamkonzentration im steady state
- C_{ss min}: Minimale Plasmakonzentration im steady state
- AUCₛₛ, 0-24: Fläche unter der Konzentrations-Zeit-Kurve (innerhalb von 24 Stunden)
- HVD: Zeit, in der Cₘₐₓ /2 gehalten wird.
- PTF: Prozentuale peak-through-Fluktuation

## Patentansprüche

1. Orale Retard-Präparation für die verzögerte Abgabe eines Wirkstoffes in Form von Tabletten unter Verwendung von magensaftresitenten Polyacrylaten, dadurch gekennzeichnet, daß eine Mischung aus pH-abhängig und pH-unabhängig retardierenden Polyacrylaten in der Matrix enthalten ist, wobei 10 bis 60 Gew.% pH-abhängig und 3 bis 30 Gew.% pH-unabhängig retardierendes Polyacrylat im Präparat vorliegt und in der Matrix als Wirkstoff Morphin, Diltiazem, Codein, Dihydrocodein, Dramatol oder Propoxyphen vorliegt.

2. Orale Retard-Präparation nach Anspruch 1, dadurch gekennzeichnet, daß 2 bis 20 Gew.% Hydroxypropylmethylcellulose im Präparat vorliegt.

3. Orale Retard-Präparation nach Anspruch 2, dadurch gekennzeichnet, daß 3 bis 6 Gew.% Hydroxypropylmethylcellulose im Präparat vorliegt.

4. Verfahren zur Herstellung eines oralen Retard-Präparates nach einem der Ansprüche 1 bis 3, dadurch gekennzeichnet, daß zu dem aus Wirkstoff, ggf. Füllstoff und Granulierflüssigkeit erhaltenen Granulat 10 % bis 60 % pH-abhängig retardierendes Polyacrylat, 3 % bis 30 % pH-unabhängig retardierendes Polyacrylat, und 2 % bis 20 % Hydroxypropylmehtylcellulose zugemischt werden, worauf unter Verwendung üblicher Tablettierhilfsstoffe zu Tabletten verpreßt wird.

5. Verfahren nach Anspruch 4, dadurch gekennzeichnet, daß die verpreßten Tabletten mit einem rasch zerfallenden Film überzogen werden.

6. Verfahren nach Anspruch 4 oder 5, dadurch gekennzeichnet, daß das Verhältnis pH-unbhängig retardierendes zu pH-abhängig retardierendem Polyacrylat so eingestellt wird, daß in der Zeiteinheit im Magen- und im Darmtrakt die gleiche Wirkstoffmenge resorbiert wird.

## Claims

1. An oral retardant preparation for the sustained release of an active ingredient in the form of tablets using gastric juice-resistant polyacrylates, characterized in that a mixture of pH-dependently and pH-independently retarding polyacrylates is contained in the matrix, wherein 10 to 60 % by weight of pH-dependently, and 3 to 30 % by weight of pH-independently, retarding polyacrylates are present in the preparation and morphine, diltiazem, codeine, dihydrocodeine, dramatol or propoxyphene is present in the matrix as the active ingredient.

2. An oral retardant preparation according to claim 1, characterized in that 2 to 20 % by weight of hydroxypropylmethyl cellulose is present in the preparation.

3. An oral retardant preparation according to claim 2, characterized in that 3 to 6 % by weight of hydroxypropoylmethyl cellulose is present in the preparation.

4. A process for preparing an oral retardant preparation according to one of claims 1 to 3, characterized in that 10 to 60 % of pH-dependently retarding polyacrylate, 3 % to 30 % of pH-independently retarding polyacrylate, and 2 % to 20 % of hydroxypropylmethyl cellulose are admixed to the granulates obtained from the active ingredient, optionally filler and granulating liquid, whereupon tablets are pressed using conventional tabletting adjuvants.

5. A process according to claim 4, characterized in that the pressed tablets are coated with a rapidly decomposing film.

6. A process according to claim 4 or 5, characterized in that the ratio of pH-independently retarding polyacrylate to pH-dependently retarding polyacrylate is adjusted in a manner that equal amounts of ingredient are resorbed in the gastrointestinal tract per time unit.

## Revendications

1. Préparation orale à effet retard pour l'administration étalée dans le temps d'une substance active sous forme de comprimés faisant appel à des polyacrylates résistants au suc gastrique, caractérisée en ce que la matrice contient un mélange de polyacrylates à effet retard dépendants et indépendants du pH, où l'on trouve dans la préparation de 10 à 60% en poids de polyacrylate à effet retard dépendant du pH et de 3 à 30% en poids de polyacrylate à effet retard indépendant du pH, et en ce que la matrice contient comme substance active de la morphine, du diltiazem, de la codéille, de la dihydrocodéine, du dramatol ou du propoxyphène.

2. Préparation orale à effet retard selon la revendication 1, caractérisée en ce que la préparation contient de 2 à 20% en poids d'hydroxyropylméthylcellulose.

3. Préparation orale à effet retard selon la revendication 2, caractérisée en ce que la préparation contient de 3 à 6% en poids d'hydroxyropylméthylcellulose.

4. Procédé de fabrication d'une préparation orale à effet retard selon l'une des revendications 1 à 3, caractérisé en ce qu'on mélange dans le granulé constitué de la substance active, le cas échéant d'une charge et d'un liquide de granulation, de 10 à 60% de polyacrylate à effet retard dépendant du pH, de 3 à 30% de polyacrylate à effet retard indépendant du pH, et de 2 à 20% d'hydroxypropylméthylcellulose, puis en ce qu'on comprime pour obtenir des comprimés en utilisant des adjuvants de compression habituels.

5. Procédé selon la revendication 4, caractérisé en ce qu'on revêt les comprimés pressés d'une pellicule qui part rapidement.

6. Procédé selon la revendication 4 ou 5, caractérisé en ce qu'on règle le rapport du polyacrylate à effet retard indépendant du pH au polyacrylate à effet retard dépendant du pH de manière que dans une unité de temps la même quantité de substance active soit absorbée dans l'estomac et dans l'intestin.
